# EUROPEAN PATENT APPLICATION

(11) **EP 1 103 624 A1**
(43) Date of publication of application: **30.05.2001**
(21) Application number: 00931703.3
(22) Date of filing: 05.06.2000
(51) Int. Cl.: C12Q 1/68, C12N 15/09, G01N 33/542, G01N 33/566

(54) **POTENTIATED NUCLEIC ACID AMPLIFICATION METHOD**

(30) Priority: 04.06.1999 JP 15765399
(71) Applicant: Tosoh Corporation, Shinnanyo-shi, Yamaguchi-ken, 746-8501 (JP)
(72) Inventor: ISHIZUKA, Tetsuya, Yokohama-shi, Kanagawa 227-0046 (JP); ISHIGURO, Takahiko, Yokohama-shi, Kanagawa 222-0034 (JP); SAITOH, Juichi, Kanagawa 242-0028 (JP); SAKAI, Tomomi, Kanagawa 243-0413 (JP)
(74) Representative: VOSSIUS & PARTNER
(86) International application number: JP0003647
(87) International publication number: WO0075371

(57) **Abstract**

A method of amplifying a specific nucleic acid for assay of the specific nucleic acid anticipated in a sample by an RNA amplification procedure which comprises forming a double-stranded DNA which contains sequences complementary and homologous to the specific RNA sequence and has a promoter sequence enabling transcription of the sequence by using the target RNA as the template and forming by using an RNA polymerase an RNA transcript which acts as the template for formation of a new single-stranded DNA, wherein in the RNA amplification procedure, inosine triphosphate is added in addition to adenosine triphosphate, uridine triphosphate, cytidine triphosphate and guanosine triphosphate to improve the efficiency of the amplification reaction.

## Description

### TECHNICAL FIELD

The present invention relates to a method of amplifying a target RNA containing a specific base sequence anticipated in a gene mixture such as DNA and RNA. In particular, it relates to a method of qualitative or quantitative assay of a target RNA which comprises amplification of the target RNA. The present invention is useful in gene diagnosis and other areas of clinical diagnostics and in identification or quantification of microorganisms in food, rooms, soil, rivers and sea.

### BACKGROUND ART

In diagnosis of virus infections, amplification of the target nucleic acid (viral nucleic acid) mostly present in trace amounts in clinical samples is generally carried out to increase signal intensity for higher sensitivity as an approach to sensitive and reproducible assay. A typical technique known for amplification of a specific DNA sequence in nucleic acid is PCR (polymerase chain reaction), which repeats thermal denaturation, primer-annealing and elongation reaction in cycles by using a couple of primers, one of which is complementary to either end of the specific sequence and the other is homologous to the other end of the specific sequence, in combination with a thermostable DNA polymerase to amplify the specific DNA sequence.

NASBA or 3SR is known as a technique for amplifying an RNA containing a specific nucleic acid (a target nucleic acid) by activating the chain reaction comprising synthesis of a double-stranded DNA having a promoter sequence from the target RNA by using a primer having a promoter sequence, reverse transcriptase and ribonuclease H, and formation by an RNA polymerase of an RNA having the specific base sequence, which is then used as the template for synthesis of the above-mentioned double-stranded DNA having the promoter sequence.

### DISCLOSURE OF THE INVENTION

In general, PCR-based RNA amplification requires a technique so-called RT-PCR, which essentially comprises two steps, preliminary reverse transcription of the template RNA into a cDNA and subsequent PCR. In addition, there is a problem that PCR is difficult to automate for a large throughput because of the need for rapid heating and cooling by a special incubator.

On the other hand, the relatively isothermal reactions involved in the above-mentioned RNA amplification technique are easy to automate. However, further improvement in the amplification efficiency is needed for sensitive and reproducible detection of a very sparse the target RNA in samples.

The present invention provide a method of amplifying a target RNA in samples with higher efficiency. The present invention also provides a method of assay of a target nucleic acid in samples based on the efficient amplification of the target RNA.

The present inventors have discovered that in the RNA amplification procedure using an RNA polymerase, the presence of a specific amount of inosine triphosphate (hereinafter referred to as "ITP") in addition to the ribonucleoside triphosphates (adenosine triphosphate, uridine triphosphate, cytidine triphosphate and guanosine triphosphate) as the substrates of the RNA polymerase increases the amplification efficiency and accomplished the present invention based on this discovery.

The invention defined in Claim 1 of the present application provides a method of amplifying a target RNA containing a specific base sequence in a sample by an RNA amplification procedure which comprises a step of forming a double-stranded DNA which has a promoter sequence and is capable of being transcribed into an RNA consisting of the specific base sequence or a sequence complementary to the specific base sequence by using the target RNA as the template, a step of forming an RNA transcript consisting of the specific base sequence or a sequence complementary to the specific base sequence by using an RNA polymerase and a step of forming the double-stranded DNA by using the RNA transcript as the template, in the presence of adenosine triphosphate, uridine triphosphate, cytidine triphosphate, guanosine triphosphate and ITP as the substrates of the RNA polymerase.

The invention defined in Claim 2 of the present application provides the amplification method according to Claim 1, wherein RNA polymerase from phage SP6 is used as the RNA polymerase, and inosine triphosphate is added to a final concentration of from 0.5 mM to 2 mM in the amplification procedure. The invention defined in Claim 3 of the present application provides the amplification method according to Claim 2, wherein the ratio of the final concentration of inosine triphosphate to the final concentration of the other ribonucleoside triphosphates (adenosine triphosphate, uridine triphosphate, cytidine triphosphate and guanosine triphosphate) is from 0.5:1.0 to 1.5:1.0.

The invention defined in Claim 4 of the present application provides the amplification method according to Claim 1, wherein in the amplification procedure, tris-HCl buffer (pH 8.5-8.9) is present at a final concentration of from 20 mM to 50 mM, magnesium chloride is present at a final concentration of from 12 mM to 20 mM, ribonucleoside triphosphates (adenosine triphosphate, uridine triphosphate, cytidine triphosphate and guanosine triphosphate) are present at a final concentration of 3.5 mM to 5.0 mM, RNA polymerase from phase T7 is present as the RNA polymerase, and inosine triphosphate is present at a final concentration of from 1.0 mM to 2.7 mM. The invention defined in Claim 5 of the present application provides the amplification method according to Claim 4, wherein the ratio of the final concentration of inosine triphosphate to the final concentration of the other ribonucleoside triphosphates (adenosine triphosphate, uridine triphosphate, cytidine triphosphate and guanosine triphosphate) is from 0.3:1.0 to 0.7:1.0.

The invention defined in Claim 6 of the present application provides the amplification method according to Claim 1, wherein in the amplification procedure, tris-HCl buffer (pH 8.5-8.9) is present at a final concentration of from 50 mM to 80 mM, magnesium chloride is present at a final concentration of from 12 mM to 20 mM, ribonucleoside triphosphates (adenosine triphosphate, uridine triphosphate, cytidine triphosphate and guanosine triphosphate) are present at a final concentration of 2.0 mM to 3.5 mM, RNA polymerase from phage T7 is present as the RNA polymerase, and inosine triphosphate is present at a final concentration of from 3.2 mM to 4.4 mM. The invention defined in Claim 7 of the present application provides the amplification method according to Claim 6, wherein the ratio of the final concentration of inosine triphosphate to the final concentration of the other ribonucleoside triphosphates (adenosine triphosphate, uridine triphosphate, cytidine triphosphate and guanosine triphosphate) is from 1.0:1.0 to 1.0:1.5.

The invention defined in Claim 8 of the present application provides the amplification method according to Claim 1, wherein the RNA amplification procedure uses a primer complementary to the specific base sequence and a primer homologous to the specific base sequence (either of which is a promoter primer having a promoter sequence for the RNA polymerase at the 5' end) and is characterized in that the target RNA is used as the template to form a single-stranded DNA by the action of an RNA-dependent DNA polymerase, the single-stranded DNA is used as the template for formation of a double-stranded DNA which has a promoter sequence and is capable of being transcribed into an RNA having the specific base sequence or a sequence complementary to the specific base sequence by the action of a DNA-dependent DNA polymerase, the double-stranded DNA is transcribed into an RNA transcript in the presence of the RNA polymerase, and the RNA transcript is used as the template for the subsequent formation of a single-stranded DNA by the RNA-dependent DNA polymerase.

The invention defined in Claim 9 of the present application provides a method of assay of a target nucleic acid which comprises carrying out the amplification procedure as defined in Claim 1 in the presence of a probe labeled with a fluorescent intercalative dye, and monitoring the fluorescence intensity of the reaction solution. The invention defined in Claim 10 of the present application provides the method according to Claim 9, wherein the probe labeled with a fluorescent intercalative dye alters its fluorescence upon hybridization with the RNA transcript.

### BRIEF DESCRIPTION OF THE DRAWINGS

Figure 1 shows the chemical structure of the fluorescent intercalative dye moiety in the fluorescent intercalative dye-labeled oligonucleotide used in Examples 1 to 4. B₁ to B₃ are nucleic acid bases.
Figure 2 is a graph correlating the reaction time and the relative fluorescence intensity at final ITP concentrations of from 0.1 mM to 1.0 mM at an initial RNA amount of 10⁶ copies/30 µℓ in Example 1. Nega indicates a sample prepared by using a diluent instead of the RNA sample.
Figure 3 is a graph correlating the reaction time and the fluorescence enhancement at final ITP concentrations of from 1.0 mM to 2.0 mM at an initial RNA amount of 10⁴ copies/30 µℓ in Example 1. Nega indicates a sample prepared by using a diluent instead of the RNA sample.
Figure 4 is a graph correlating the reaction time and the fluorescence enhancement at final ITP concentrations of from 0.5 mM to 3.0 mM at an initial RNA amount of 10⁶ copies/30 µℓ in Example 2. Nega indicates a sample prepared by using a diluent instead of the RNA sample.
Figure 5 is a graph correlating the reaction time at detection of the specific product by fluorescent dye-labeled oligonucleotides (YO-271 and YO-WT) complementary to two RNA samples (HCV and WQ) respectively, and the relative fluorescence intensity at an initial RNA amount of 10⁵ copies/30 µℓ in Example 3 . Nega indicates a sample prepared by using a diluent instead of the RNA sample. Only the specific combinations (HCV with YO-271 and WQ with YO-WT) gave fluorescence enhancement.
Figure 6 is a negative photograph showing the results of 4% agarose gel electrophoresis of samples after the reaction in Example 3. Samples containing the RNA sample (lanes 1, 2, 4 and 5) only gave distinctive bands. The samples in lanes 7 and 8 were authentic samples of HCV-RNA (155 bp) and WQ-RNA (102 bp), respectively. The molecular marker was φX174/HaeIII.
Figure 7 is a graph correlating the reaction time and the fluorescence enhancement at final ITP concentrations of from 2.8 mM to 4.4 mM at an initial RNA amount of 10⁵ copies/30 µℓ in Example 4. Nega indicates a sample prepared by using a diluent instead of the RNA sample.
Figure 8 shows the rising time (the relative fluorescence intensity at final ITP concentrations of from 2.8 mM to 4.4 mM at an initial RNA amount of 10⁵ copies/30 µℓ in Example 4: Nega indicates a sample prepared by using a diluent instead of the RNA sample.

### BEST MODE FOR CARRYING OUT THE INVENTION

The present invention is based on the discovery that ITP improves the efficiency of RNA synthesis by an RNA polymerase along a template DNA.

The present invention can be carried out, for example, by adding the following reagents (A) to (I) on hand one by one, in combinations of at least two or all at once (in any order):
(A) a first single-stranded oligonucleotide complementary to a 3'-end sequence of the specific base sequence in the single-stranded RNA,
(B) an RNA-dependent DNA polymerase,
(C) deoxyribonucleoside triphosphates,
(D) a ribonuclease H or an enzyme having similar RNA degrading activity,
(E) a second single-stranded oligonucleotide having (1) a promoter sequence for a DNA-dependent RNA polymerase, (2) an enhancer sequence for the promoter and (3) a 5'-end sequence of the specific base sequence in the single-stranded RNA, in this order from the 5' end,
(F) a DNA-dependent DNA polymerase,
(G) a DNA-dependent RNA polymerase,
(H) ribonucleoside triphosphates (adenosine triphosphate, uridine triphosphate, cytidine triphosphate and guanosine triphosphate), and
(I) ITP.

Instead of the combination of the first and second single-stranded oligonucleotides defined above as the reagents (A) and (E), the combination of the first and second single-stranded oligonucleotides defined below as the reagents (J) and (K) may be used:
(J) a first single-stranded oligonucleotide having (1) a promoter sequence for a DNA-dependent RNA polymerase, (2) an enhancer sequence for the promoter and (3) a sequence complementary to a 3'-end sequence of the specific base sequence in the target RNA, in this order from the 5' end, and
(K) a second single-stranded oligonucleotide homologous to a 5'-sequence of the specific base sequence in the target RNA.
   When the first and second single-stranded oligonucleotides defined above as the reagents (A) and (E) are used, the above-mentioned specific base sequence means part of the base sequence of the target RNA which starts with the sequence (3) in the second single-stranded oligonucleotide as the reagent (E) from the 5' end and ends with a sequence complementary to the first single-stranded oligonucleotide as the reagent (A) at the 3' end. When the first and second single-stranded oligonucleotides defined above as the reagents (J) and (K) are used, the above-mentioned specific base sequence means part of the base sequence of the target RNA which starts with the sequence homologous to the second single-stranded oligonucleotide as the reagent (K) from the 5' end and ends with a sequence complementary to the sequence (3) in the first single-stranded oligonucleotide as the reagent (J) at the 3' end. The specific sequence may be selected arbitrary, but should include a sequence specific enough to distinguish the target RNA from other nucleic acids.
   The reagent (A) is a first single-stranded oligonucleotide complementary to a 3'-end sequence of the specific base sequence. It hybridizes with the target RNA and makes sure of formation of a cDNA having a 5' end complementary to the 3'-end of the specific base sequence along the target RNA as the template by the action of an RNA-dependent DNA polymerase in the presence of the reagents (B) and (C) mentioned later.
   The reagent (B) is an RNA-dependent DNA polymerase, and the reagent (C) is its substrate, deoxyribonucleotide triphosphates. A cDNA having a 5' end complementary to the 3' end of the specific base sequence in the target RNA is synthesized in the presence of the reagents (A) to (C). The cDNA is in the form of a DNA-RNA double strand with the template RNA.
   The reagent (D) is a ribonuclease H having an activity of cleaving the RNA in the above-mentioned DNA-RNA double strand or may be an enzyme having a similar RNA degrading activity. Reverse transcriptases such as avian myoblastoma virus polymerase (hereinafter AMV reverse transcriptase) have an activity of cleaving the RNA in the above-mentioned DNA-RNA double strand, and enzymes of this type may, for example, be used.
   The reagent (E) is a second single-stranded oligonucleotide having (1) a promoter sequence for a DNA-dependent RNA polymerase, (2) an enhancer sequence for the promoter and (3) a 5'-end sequence of the specific base sequence in the single-stranded RNA, in this order from the 5' end. The moiety (3) in the second oligonucleotide binds to the 3'-end of the cDNA synthesized in the presence of the reagents (A) to (D) to induce elongation of the second oligonucleotide from the 3' end by using the cDNA as the template and simultaneous elongation of the cDNA from the 3' end using the second oligonucleotide as the template by a DNA-dependent DNA polymerase in the presence of the reagents (C) and (F). Thus, the second oligonucleotide and the cDNA complement each other, forming a transcribable fully double-stranded DNA having a promoter sequence.
   The reagent (F) is a DNA-dependent DNA polymerase or may be a reverse transcriptase having a DNA-dependent DNA polymerase activity represented by AMV reverse transpriptase.
   The reagent (G) is a DNA-dependent RNA polymerase, and the reagents (H) and (I) are its substrates, ribonucloside triphosphates and ITP. The double-stranded DNA synthesized in the presence of the reagents (C), (E) and (F) has a segment which acts as a promoter for a DNA-dependent RNA polymerase at either end. Therefore, in the presence of the reagents (G) and (H), once the DNA is synthesized, synthesis of a single-stranded RNA having the specific base sequence starts immediately. The presence of the reagent (I) dramatically increases the yield of the RNA, as described above. Specific examples of the DNA-dependent RNA polymerase as the reagent (G) include T7 RNA polymerase and SP6 RNA polymerase.
   The single-stranded RNA synthesized in the presence of the reagents (G) to (I) is an RNA having the specific base sequence (partly substituted by ITP). Therefore, the presence of the reagents (A) to (F) with the RNA thus synthesized allows the above-mentioned series of reactions to recur. Thus, in the present invention, a trace of the target RNA in a sample acts as the starter in synthesis the above-mentioned double-stranded DNA having a promoter region, and in turn, the double-stranded DNA acts as the starter in synthesis of a single-stranded RNA having the specific base sequence. The resulting single-stranded RNA contributes to synthesis of a new double-stranded DNA. Consequently, the amount of the single-stranded RNA having the specific base sequence increases dramatically with time.
   The present invention also provides a method of assay a target RNA which comprises carrying out the above-mentioned RNA amplification procedure in the presence of the reagent (L), and monitoring the fluorescence intensity of the reaction solution:
(L) a third single-stranded oligonucleotide complementary to the specific base sequence which is so labeled to give off a measurable signal upon binding to a nucleic acid having the specific base sequence.
   The third single-stranded oligonucleotide is, for example, a DNA linked to a fluorescent intercalative dye. The DNA moiety is 6 to 100 nucleotides long, preferably 10 to 30 nucleotides long, Of course, the DNA moiety must be complementary to part of the specific base sequence which sufficiently distinguish the target nucleic acid from other nucleic acids. It is preferred that the DNA moiety has a 3' end uncomplimentary to the specific base sequence or a chemically modified 3' end so that it does not elongate from the 3' end by the action of the RNA-dependent DNA polymerase previously added as the reagent (C) upon hybridization with the synthesized single-stranded RNA having the specific base sequence.
   If the DNA moiety hybridizes with another nucleic acid, the fluorescent intercalative dye intercalates into the resulting double strand and changes its fluorescence characteristic. For this purpose, the fluorescent intercalative dye may be linked to the DNA moiety via a linker of an appropriate length. Any linker that does not hinder the fluorescent intercalative dye from intercalating into the double strand may be used without any particular restriction. A bifunctional hydrocarbon linker having functional groups at both ends is particularly preferable in view of the ease of the modification of oligonucleotides for its use. Alternatively, a reagent commercial kit (C6-Thiolmodifier, tradename, Clonntech) may be used.
   The fluorescent intercalative dye is not particularly limited as long as it changes the fluorescent characteristic, for example emits fluorescence having a different peak wavelength, on intercalation into a double strand. However, those which enhance the fluorescence on intercalation are particularly preferable in view of the ease of fluorescence measurement. More specifically, particularly preferable fluorescent intercalative dyes are, for example, thiazole orange, oxazole yellow and their derivatives, because they show radical change in the fluorescence.
   The fluorescent intercalative dye may be linked to any sites of the DNA moiety, including the 5' end, the 3' end and the middle, as long as the linkage neither hinders the fluorescent intercalative dye from intercalating into a double strand nor hinders the DNA moiety from hybridizing with RNA.
   The amount of the single-stranded RNA synthesized along the template double-stranded DNA having a promoter sequence in the presence of the reagents (G) to (I) increases with time. Coexistence of the reagent (L) with at least the reagents (A) to (I) in a sample anticipated to contain the target RNA enables determination of the increasing single-stranded RNA having the specific base sequence by monitoring the fluorescent signal. The synthesized single-stranded RNA has been found to serve as a template for DNA synthesis in the presence of the reagents (A) to (C) even when the hybrid of the RNA and the third oligonucleotide as the reagent (L) is giving off a fluorescent signal. In other words, in the present invention, a series of events, synthesis of a cDNA from an RNA, synthesis of a double-stranded DNA and synthesis of an RNA from the double-stranded DNA in the presence of the respective reagents, take place in the presence of the third oligonucleotide with increase in the fluoresce intensity proportional to increase of the RNA.
   In the present invention, when the reagents (J) and (K) are used instead of the reagents (A) and (E), the following reagent (M) should be used instead of the reagent (L) for assay of a specific nucleic acid:
(M) a third single-stranded oligonucleotide homologous to the specific base sequence which is so labeled to give off a measurable signal upon binding to a nucleic acid complementary to the specific base sequence.
   The reagent (J) is a first single-stranded oligonucleotide having (1) a promoter sequence for a DNA-dependent RNA polymerase, (2) an enhancer sequence for the promoter and (3) a sequence complementary to a 3'-end sequence of the specific base sequence in the target RNA, in this order from the 5' end. The moiety (3) in the reagent hybridizes with the 3'-end of the specific acid sequence to form a cDNA complementary to the target RNA in the presence of the reagents (B) and (C) and (D). The reagent (K) is a single-stranded oligonucleotide complementary to a 5'-end sequence of the specific base sequence in the cDNA. The reagent (K) hybridizes with the 5' end of the specific sequence in the CDNA to form a double-stranded DNA having the promoter sequence for an RNA polymerase in the presence of the reagents (C) and (F). The double-stranded DNA is, in turn, transcribed into a second single-stranded RNA complementary to the specific base sequence in the presence of the reagents (G) to (H). The presence of the reagent (I) increases the yield of the single-stranded RNA dramatically.
   The 3' end of the second RNA hybridizes with the reagent (K) to form a second cDNA complementary to the second RNA in the presence of the reagents (B) to (D). The second cDNA hybridizes with the moiety (3) in the reagent (J) to form a double-stranded DNA having the promoter sequence in the presence of the reagents (C) and (F), and then the second RNA is synthesized in the presence of the reagents (G) to (I). The resulting second RNA complementary to the specific base sequence acts as the starter in synthesis of a new double-stranded DNA. Such repetition of a series of reactions ends in dramatic increase of the second RNA.
   The second RNA thus synthesized is complementary to the specific base sequence in the target RNA. By virtue of the presence of the third single-stranded oligonucleotide as the reagent (M) which is homologous to the specific base sequence and labeled with a fluorescent intercalative dye, the fluorescence intensity increases proportionally to the yield of the second RNA.
   In the present invention, the addition of the reagents (A) to (I) and the reagent (L) or (M) is preferably followed by durational measurement of fluorescent signals immediately or after a certain time lag. Though during synthesis of the single-stranded RNA, the third DNA as the reagent (L) or (M) binds to and separates from the synthesized RNA repeatedly, but it is possible to monitor the increase of the single-stranded RNA because the measured fluorescent signals from the bound third DNA reflects the amounts of the RNA at the moments of the measurements. The measurement may be done continuously or intermittently at constant intervals. The instrument for measuring the fluorescent signal may be of any type so long as it can measure the fluorescent signal from at least one reaction tube continuously or intermittently at constant intervals, though it does not constitute part of the present invention.

The fluorescence measurement is carried out over about 4 hours, preferably 1 hour until the fluorescence intensity from a sample containing a certain amount of the target RNA nearly levels off so as to cover the stage which marks a significant difference in fluorescence intensity enhancement between a sample containing a certain amount of the target RNA and a sample free of the target RNA.

Now, the present invention will be described in further details by referring to Examples. However, the present invention should be by no means is restricted to these specific Examples.

### EXAMPLE 1

The effect of ITP (final concentration 0.1 mM to 2.0 mM) on amplification of a target nucleic acid was investigated.
(1) The standard RNA sample, the bases 113-267 of human hepatitis C virus RNA (Kato et al,. Proc. Natl. Sci., USA, 1990, 87, 9524-9528), was determined from ultraviolet absorption at 260 nm and then diluted with an RNA diluent (10 mM Tris-HCl (pH 8.0), 0.1 mM EDTA, 0.5 U/µℓ RNase Inhibitor) to 10⁶ copies and 10⁴ copies/4 µℓ. For control samples (Nega), only the RNA diluent was used.
(2) 15.2 µℓ portions of a reaction solution of the following composition was dispensed into 0.5 mℓ commercial PCR tubes (trade name; Gene Amp Thin-Walled Reaction Tubes, Perkin Elmer) and after addition of 4 µℓ of the RNA sample, overlaid with 100 µℓ of mineral oil.
The composition of the reaction solution

| | |
|---|---|
| 59.2 mM | Tris-acetate buffer (pH 8.1) |
| 13.2 mM | Magnesium acetate |
| 123.7 mM | Potassium acetate |
| 15.8% | Sorbitol |
| 19.7 mM | DTT |
| 1.0 mM | each of dATP, dCTP, dGTP and dTTP |
| 2.0 mM | each of ATP, CTP, GTP and UTP |
| Various concentrations (0.2 mM, 1.0 mM, 2.0 mM, 3.0 mM and 4.0 mM) | ITP |
| 0.4 µM | First oligonucleotide (SEQ ID NO:1) |
| 0.4 µM | Second oligonucleotide having SP6 promoter sequence (SEQ ID NO:2; comprising SP6 promoter sequence from "A" at position 1 from the 5' end to "A" at position 17 and an enhancer sequence from "G" at position 18 to "A" at position 25) |
| 0.049 µM | Third oligonucleotide labeled with a fluorescent intercalative dye (Fig. 1) (SEQ ID NO:3; having the fluorescent intercalative dye between "C" at position 5 and "G" at position 6 as the label and having a 3'-end modification with glycolic acid, hereinafter referred to simply as YO-271) |
| 60 units | Ribonuclease inhibitor (Takara Shuzo Co., Ltd.) |
| 3.9% | DMSO |
| Distilled water for volume adjustment | |

(3) The reaction solutions were incubated at 50°C for 5 minutes, and 10.8 µℓ of an enzyme solution of the following composition was added.
The composition of the enzyme solution

| | |
|---|---|
| 83.3 mM | Tris-acetate buffer (pH 8.1) |
| 18.5 mM | Magnesium acetate |
| 174.1 mM | Potassium acetate |
| 22.2% | Sorbitol |
| 42 units | AMV reverse transcriptase (Takara Shuzo Co., Ltd.) |
| 171 units | SP6 RNA polymerase (Takara Shuzo Co., Ltd.) |
| 3 µg | Bovine serum albumin |
| Distilled water for volume adjustment | |

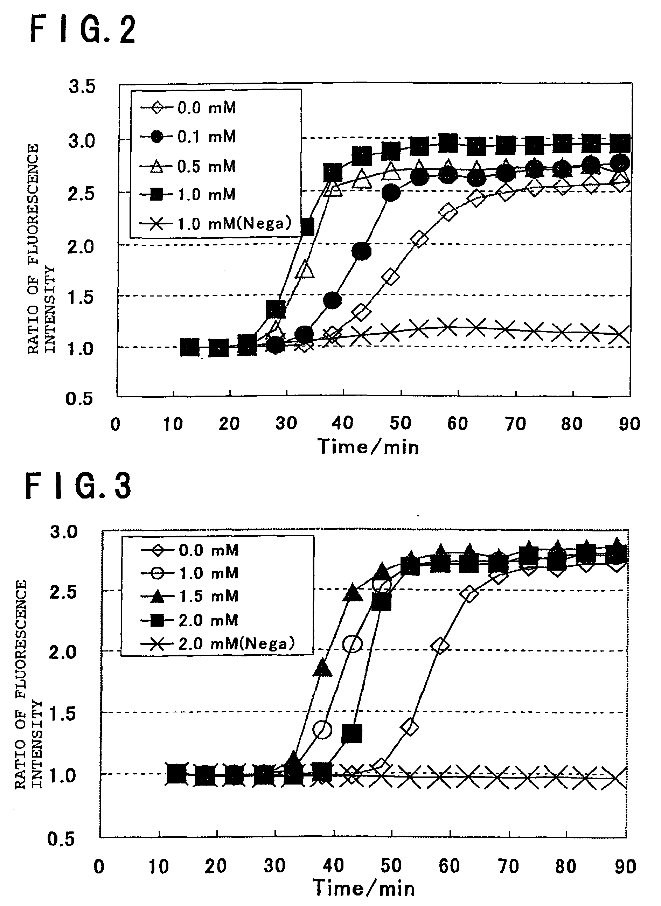

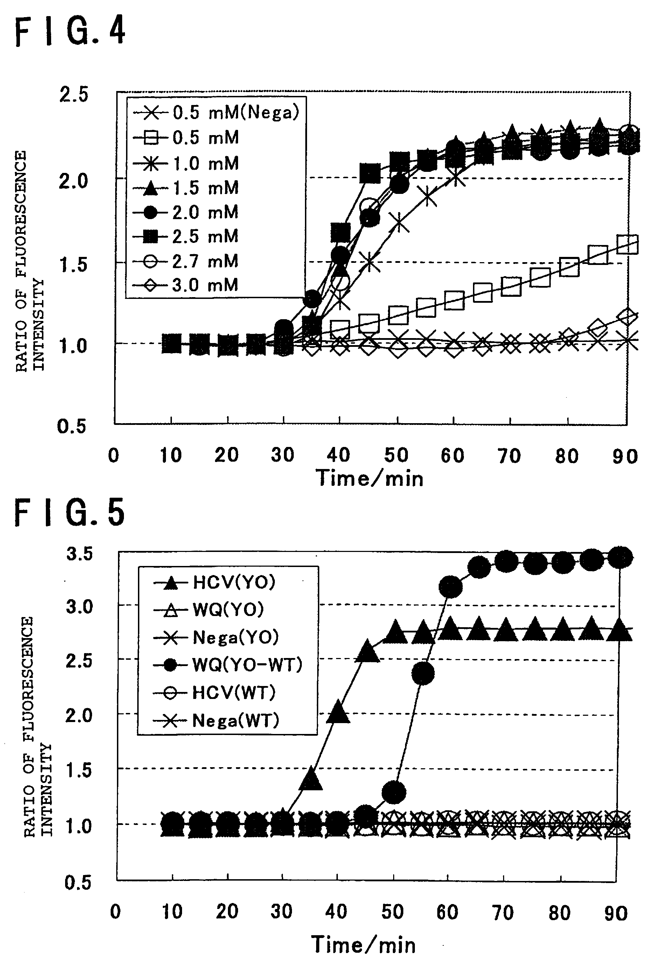

(4) The fluorescence intensities of the reaction solutions in the PCR tubes were measured at 50°C at a 5-minute intervals in a thermostatic fluorescent spectrophotometer at an excitation wavelength of 490 nm and an emission wavelength of 510 nm.
The time courses of the ratio of fluorescence intensities of the samples (fluorescence intensity at a certain time/background fluorescence intensity) from addition of the enzyme solution at 0 minute were shown in Fig. 2 (RNA sample concentration 10⁶ copies/test, ITP final concentration 0.1 mM-1.0 mM) and in Fig. 3 (RNA sample concentration 10⁴ copies/test, ITP final concentration 1.0 mM-2.0 mM).
The results indicate that with SP6 RNA polymerase as the RNA polymerase, the addition of ITP at final concentrations of from 0.5 mM-2 mM to the reaction system dramatically improved the efficiency of amplification of the specific nucleic acid. Because no increase in the fluorescence intensity was observed with negative samples (Nega), the increase in fluorescence intensity seemed to reflect the increase of the specific base sequence.

### EXAMPLE 2

The effect of addition of ITP (final concentration 0.5 mM to 3.0 mM) on amplification of a target nucleic acid was investigated.
(1) The above-mentioned human hepatitis C virus RNA as the standard RNA sample was determined from ultraviolet absorption at 260 nm and then diluted with an RNA diluent (10 mM Tris-HCl (pH 8.0), 0.1 mM EDTA, 0.5 U/µℓ RNase Inhibitor) to 10⁶ copies/2.5 µℓ. For control samples (Nega), only the RNA diluent was used.
(2) 21.0 µℓ portions of a reaction solution of the following composition was dispensed into 0.5 mℓ commercial PCR tubes (trade name; Gene Amp Thin-Walled Reaction Tubes, Perkin Elmer) and after addition of 2.5 µℓ of the RNA sample, overlaid with 100 µℓ of mineral oil.
The composition of the reaction solution

| | |
|---|---|
| 47.6 mM | Tris-HCl buffer (pH 8.5) |
| 17.1 mM | Magnesium chloride |
| 185.7 mM | Potassium chloride |
| 1.4 mM | DTT |
| 0.7 mM | each of dATP, dCTP, dGTP and dTTP |
| 5.4 mM | each of ATP, CTP, GTP and UTP |
| 0.7, 1.4, 2.1, 2.9, 3.6, 3.9 or 4.3 mM | ITP |
| 1.1 µM | First oligonucleotide (SEQ ID NO:4) |
| 1.1 µM | Second oligonucleotide having T7 promoter sequence (SEQ ID NO:5; comprising T7 promoter sequence from "A" at position 1 from the 5' end to "A" at position 22 and an enhancer sequence from "G" at position 23 to "A" at position 28) |
| 0.049 µM | Third oligonucleotide labeled with a fluorescent intercalative dye (Fig. 1) (SEQ ID NO:6; having the fluorescent intercalative dye between "A" at position 14 from the 5' end and "C" at position 15 as the label and having a 3'-end modification with glycolic acid, hereinafter referred to simply as YO-HC1b) |
| 40 units | Ribonuclease inhibitor (Takara Shuzo Co., Ltd.) |
| 21.4% | DMSO |
| Distilled water for volume adjustment | |

(3) The reaction solutions were incubated at 41°C for 5 minutes, and 6.5 µℓ of an enzyme solution of the following composition was added.
The composition of the enzyme solution

| | |
|---|---|
| 30.8 mM | Tris-HCl buffer (pH 8.5) |
| 7.8% | Sorbitol |
| 29 units | AMV reverse transcriptase (Takara Shuzo Co., Ltd.) |
| 142 units | T7 RNA polymerase (Toyobo Co., Ltd) |
| 3 µg | Bovine serum albumin |
| Distilled water for volume adjustment | |

(4) The fluorescence intensities of the reaction solutions in the PCR tubes were measured at 50°C at a 5-minute intervals in a thermostatic fluorescent spectrophotometer at an excitation wavelength of 490 nm and an emission wavelength of 510 nm.

The time courses of the ratio of fluorescence intensities of the samples (fluorescence intensity at a certain time/background fluorescence intensity) from addition of the enzyme solution at 0 minute were shown in Fig. 4 (RNA sample concentration 10⁶ copies/test, ITP final concentration 0.5 mM-3.0 mM).

The results indicate that with T7 RNA polymerase as the RNA polymerase, ITP improved the efficiency of amplification of the specific nucleic acid more dramatically at final concentrations of 1.0 mM-2.7 mM than at a final concentration of 0.5 mM. The detection of the RNA having the specific base sequence proved successful because no increase in the fluorescence intensity was observed with negative samples (Nega).

### EXAMPLE 3

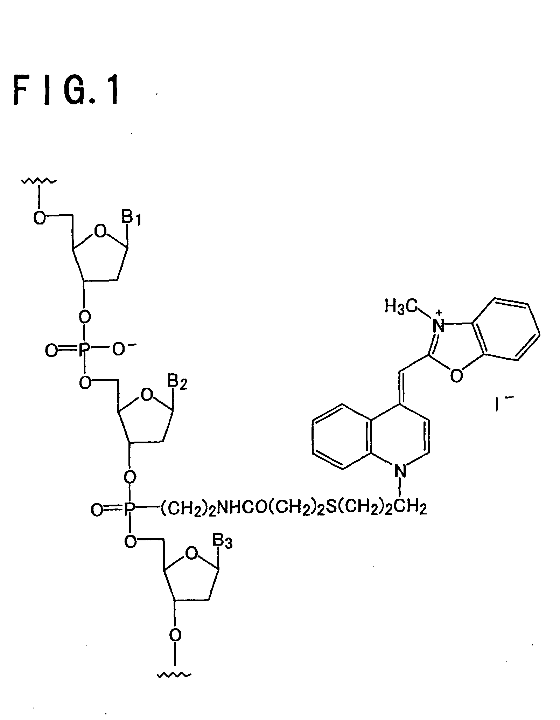

The detection of specific products with a third oligonucleotide and a fourth oligonucleotide labeled with a fluorescent intercalative dye (Fig.1) was investigated.
(1) The above-mentioned standard RNA (HCV-RNA) and a synthetic RNA (WQ-RNA, SEQ ID NO:7) were diluted with an RNA diluent (10 mM Tris-HCl (pH 8.0), 0.1 mM EDTA, 0.5 U/µℓ RNase Inhibitor) to 10⁵ copies/4 µℓ. For control samples (Nega), only the RNA diluent was used.
(2) The following combinations of RNA samples and oligonucleotides labeled with a fluorescent intercalative dye (Fig. 1) were made in the solution prepared in (3).
RNA sample Fluorescent dye-labeled oligonucleotide

| | |
|---|---|
| ① HCV RNA | Third oligonucleotide(YO-271) |
| ② WQ-RNA | Third oligonucleotide(YO-271) |
| ③ Nega | Third oligonucleotide(YO-271) |
| ④ WQ-RNA | Fourth oligonucleotide(YO-WT) |
| ⑤ HCV RNA | Fourth oligonucleotide(YO-WT) |
| ⑥ Nega | Fourth oligonucleotide(YO-WT) |

(3) 15.2 µℓ portions of a reaction solution of the following composition were dispensed into 0.5 mℓ commercial PCR tubes (trade name; Gene Amp Thin-Walled Reaction Tubes, Perkin Elmer) and after addition of 4 µℓ of the RNA sample, overlaid with 100 µℓ of mineral oil.
The composition of the reaction solutions

| | |
|---|---|
| 59.2 mM | Tris-acetate buffer (pH 8.1) |
| 13.2 mM | Magnesium acetate |
| 123.7 mM | Potassium acetate |
| 15.8% | Sorbitol |
| 19.7 mM | DTT |
| 1.0 mM | each of dATP, dCTP, dGTP and dTTP |
| 2.0 mM | each of ATP, CTP, GTP and UTP |
| 2.5 mM | ITP |
| 0.4 µM | First oligonucleotide (SEQ ID NO:1) |
| 0.4 µM | Second oligonucleotide having SP6 promoter sequence (SEQ ID NO:2) |
| 0.049 µM | Third oligonucleotide labeled with a fluorescent intercalative dye (Fig. 1) (YO-271) or a fourth oligonucleotide labeled with a fluorescent intercalative dye (Fig. 1) (SEQ ID NO:8; having the fluorescent intercalative dye between "A" at position 10 from the 5' end and "G" at position 11 as the label and having a 3'-end modification with glycolic acid, hereinafter referred to simply as YO-WT) |
| 60 units | Ribonuclease inhibitor |
| 3.9% | DMSO |
| Distilled water for volume adjustment | |

(4) The reaction solutions were incubated at 50°C for 5 minutes, and 10.8 µℓ of an enzyme solution of the following composition was added.
The composition of the enzyme solution

| | |
|---|---|
| 83.3 mM | Tris-acetate buffer (pH 8.1) |
| 18.5 mM | Magnesium acetate |
| 174.1 mM | Potassium acetate |
| 22.2% | Sorbitol |
| 42 units | AMV reverse transcriptase (Takara Shuzo Co., Ltd.) |
| 171 units | SP6 RNA polymerase (Takara Shuzo Co., Ltd.) |
| 3 µg | Bovine serum albumin |
| Distilled water for volume adjustment | |

(5) The fluorescence intensities of the reaction solutions in the PCR tubes were measured at 50°C at a 5-minute intervals in a thermostatic fluorescent spectrophotometer at an excitation wavelength of 490 nm and an emission wavelength of 510 nm.

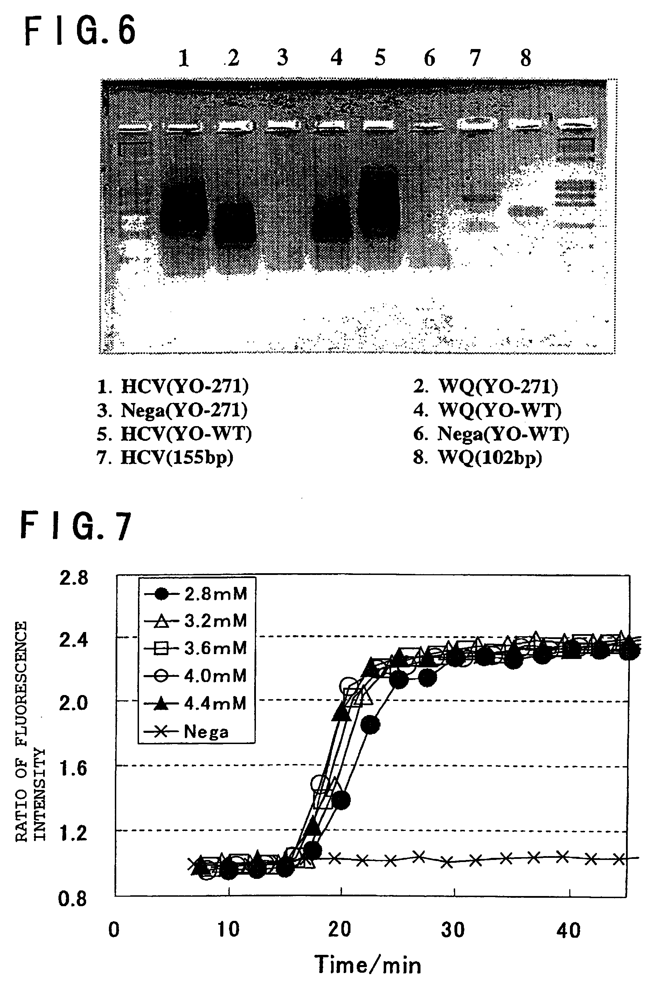

The time courses of the ratio of fluorescence intensities of the samples (fluorescence intensity at a certain time/background fluorescence intensity) from addition of the enzyme solution at 0 minute were shown in Fig. 5, and the results of electrophoresis of the samples after the amplification are shown in Fig. 6. As shown in Fig. 5, only the combinations of target nucleic acids and fluorescent dye-labeled oligonucleotides complementary to them (HCV and YO-271, and WQ and YO-WT) gave fluorescence increase. As shown in Fig. 6, distinctive bands were detected with samples containing RNA samples (lanes 1, 2, 4 and 5). These results indicate that even in the presence of ITP, the fluorescence intensities measured only reflected amplification of the specific products.

### EXAMPLE 4

The reaction conditions in Example 2 were further optimized (at final ITP concentrations between 2.8 mM-4.4 mM) to find the optimum concentration of ITP for amplification of the target nucleic acid.
(1) The standard RNA sample, recombinant RNA of 1549 nucleotides long containing the bases 1-1487 of human hepatitis C virus RNA (Kato et al,. Proc. Natl. Sci., USA, 1990, 87, 9524-9528), was determined from ultraviolet absorption at 260 nm and then diluted with an RNA diluent (10 mM Tris-HCl (pH 8.0), 0.1 mM EDTA, 0.5 U/µℓ RNase Inhibitor, 5.0 mM DTT) to 10⁵ copies/5 µℓ. For control samples (Nega), only the RNA diluent was used.
(2) 20.8 µℓ portions of a reaction solution of the following composition was dispensed into 0.5 mℓ commercial PCR tubes (trade name; Gene Amp Thin-Walled Reaction Tubes, Perkin Elmer), and 5 µℓ of the RNA sample was added.
The composition of the reaction solution (in terms of the final concentrations in the reaction system after addition of the enzyme solution)

| | |
|---|---|
| 60.0 mM | Tris-HCl buffer (pH 8.6) |
| 13.0 mM | Magnesium chloride |
| 90.0 mM | Potassium chloride |
| 1.0 mM | DTT |
| 0.25 mM | each of dATP, dCTP, dGTP and dTTP |
| 3.0 mM | each of ATP, CTP, UTP and GTP |
| 2.8, 3.2, 3.6, 4.0 or 4.4 mM | ITP |
| 1.0 µM | First oligonucleotide (SEQ ID NO:1) |
| 1.0 µM | Second oligonucleotide (SEQ ID NO:9; comprising T7 promoter sequence from "A" at position 1 from the 5' end to "A" at position 28) |
| 0.16 µM | Scissor oligonucleotide (SEQ ID NO:10; oligonucleotide for cleaving the target RNA at the 5' end of the specific sequence which is complementary to a redundant segment neighboring the 5' end of the specific sequence, forms an RNA-DNA double strand with the target RNA to direct a reverse transcriptase to degrade the RNA in the RNA-DNA double strand by exerting its RNase H activity and has an aminated 3' end not to function as an oligonucleotide primer) |
| 25.0 nM | Oligonucleotide labeled with a fluorescent intercalative dye (Fig. 1) (YO-271) (SEQ ID NO:3; having a 3'-end modification with glycolic acid) |
| 39 units | Ribonuclease inhibitor (Takara Shuzo Co., Ltd.) |
| 15.0% | DMSO |
| Distilled water for volume adjustment | |

(3) The reaction solutions were incubated at 41°C for 5 minutes, and 4.2 µℓ of an enzyme solution of the following composition which was pre-incubated at 41°C for 2 minutes was added.
The composition of the enzyme solution (in terms of the final concentrations in the reaction)

| | |
|---|---|
| 1.7% | Sorbitol |
| 8 units | AMV reverse transcriptase (Takara Shuzo Co., Ltd.) |
| 142 units | T7 RNA polymerase (GIBCO) |
| 3 µg | Bovine serum albumin |
| Distilled water for volume adjustment | |

(4) The fluorescence intensities of the reaction solutions in the PCR tubes were monitored at 41°C in a thermostatic fluorescent spectrophotometer at an excitation wavelength of 470 nm and an emission wavelength of 510 nm.

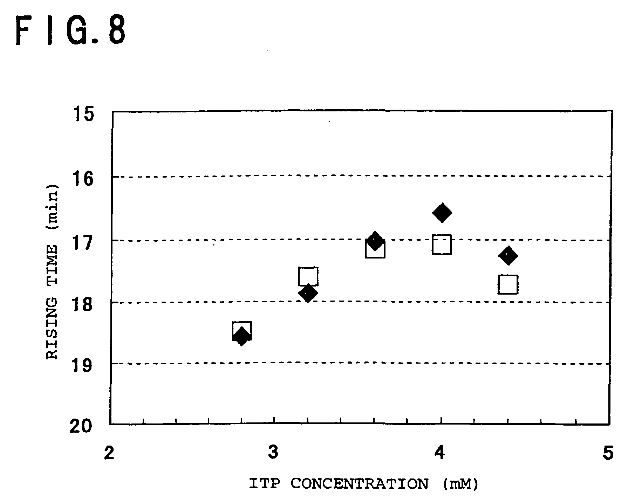

The time courses of the ratio of fluorescence intensities of the samples (fluorescence intensity at a certain time/background fluorescence intensity) from addition of the enzyme solution at 0 minute were shown in Fig. 7. The relation between the rising time when the relative fluorescence intensity surpassed 1.2 and the ITP concentration is shown in Fig. 8.

Fig. 7 and Fig. 8 indicate that the optimum final ITP concentration for the shortest rising time is between 3.2 mM and 4.4 mM. Thus, a high-efficiency amplification system in the presence of ITP which enables detection of 10⁵ copies/test of RNA in 17 minutes was established. The detection of the RNA having the specific base sequence proved successful because no increase in the fluorescence intensity was observed with negative samples (Nega).

### INDUSTRIAL APPLICABILITY

As described above, according to the present invention, addition of ITP dramatically improves the amplification efficiency of almost isothermal and speedy amplification of a specific RNA sequence originally contained in a sample which requires only one step manual operation on a single-stranded RNA containing the specific base sequence in the sample to start the reaction without repetitious rapid heating or cooling of the reaction solution.

Further, according to the present invention, the target RNA in a sample acts as the starter in synthesis the above-mentioned double-stranded DNA having a promoter region for a DNA-dependent RNA polymerase, which in turn, acts as the starter in synthesis of a large amount of a single-stranded RNA, the yield of the single-stranded RNA increases dramatically, and analysis of the time course of the measured fluorescence intensity increase resulting from the hybridization between a fluorescent intercalative dye and the single-stranded RNA provides a way to simple and rapid determination of the initial amount of the RNA.

## Claims

1. A method of amplifying a target RNA containing a specific base sequence in a sample by an RNA amplification procedure which comprises a step of forming a double-stranded DNA which has a promoter sequence and is capable of being transcribed into an RNA consisting of the specific base sequence or a sequence complementary to the specific base sequence by using the target RNA as the template, a step of forming an RNA transcript consisting of the specific base sequence or a sequence complementary to the specific base sequence by using an RNA polymerase and a step of forming the double-stranded DNA by using the RNA transcript as the template, in the presence of adenosine triphosphate, uridine triphosphate, cytidine triphosphate, guanosine triphosphate and inosine triphosphate as the substrates of the RNA polymerase.

2. The amplification method according to Claim 1, wherein RNA polymerase from phage SP6 is used as the RNA polymerase, and inosine triphosphate is added to a final concentration of from 0.5 mM to 2 mM in the amplification procedure.

3. The amplification method according to Claim 2, wherein the ratio of the final concentration of inosine triphosphate to the final concentration of the other ribonucleoside triphosphates (adenosine triphosphate, uridine triphosphate, cytidine triphosphate and guanosine triphosphate) is from 0.5:1.0 to 1.5:1.0.

4. The amplification method according to Claim 1, wherein in the amplification procedure, tris-HCl buffer (pH 8.5-8.9) is present at a final concentration of from 20 mM to 50 mM, magnesium chloride is present at a final concentration of from 12 mM to 20 mM, ribonucleoside triphosphates (adenosine triphosphate, uridine triphosphate, cytidine triphosphate and guanosine triphosphate) are present at a final concentration of 3.5 mM to 5.0 mM, RNA polymerase from phase T7 is present as the RNA polymerase, and inosine triphosphate is present at a final concentration of from 1.0 mM to 2.7 mM.

5. The amplification method according to Claim 4, wherein the ratio of the final concentration of inosine triphosphate to the final concentration of the other ribonucleoside triphosphates (adenosine triphosphate, uridine triphosphate, cytidine triphosphate and guanosine triphosphate) is from 0.3:1.0 to 0.7:1.0.

6. The amplification method according to Claim 1, wherein in the amplification procedure, tris-HCl buffer (pH 8.5-8.9) is present at a final concentration of from 50 mM to 80 mM, magnesium chloride is present at a final concentration of from 12 mM to 20 mM, ribonucleoside triphosphates (adenosine triphosphate, uridine triphosphate, cytidine triphosphate and guanosine triphosphate) are present at a final concentration of 2 mM to 3.5 mM, RNA polymerase from phage T7 is present as the RNA polymerase, and inosine triphosphate is present at a final concentration of from 3.2 mM to 4.4 mM.

7. The amplification method according to Claim 6, wherein the ratio of the final concentration of inosine triphosphate to the final concentration of the other ribonucleoside triphosphates (adenosine triphosphate, üridine triphosphate, cytidine triphosphate and guanosine triphosphate) is from 1.0:1.0 to 1.0:1.5.

8. The amplification method according to Claim 1, wherein the RNA amplification procedure uses a primer complementary to the specific base sequence and a primer homologous to the specific base sequence (either of which is a promoter primer having a promoter sequence for the RNA polymerase at the 5' end) and is characterized in that the target RNA is used as the template to form a single-stranded DNA by the action of an RNA-dependent DNA polymerase, the single-stranded DNA is used as the template for formation of a double-stranded DNA which has a promoter sequence and is capable of being transcribed into an RNA having the specific base sequence or a sequence complementary to the specific base sequence by the action of a DNA-dependent DNA polymerase, the double-stranded DNA is transcribed into an RNA transcript in the presence of the RNA polymerase, and the RNA transcript is used as the template for the subsequent formation of a single-stranded DNA by the RNA-dependent DNA polymerase.

9. A method of assay of a target nucleic acid which comprises carrying out the amplification procedure as defined in Claim 1 in the presence of a probe labeled with a fluorescent intercalative dye, and monitoring the fluorescence intensity of the reaction solution.

10. The method according to Claim 9, wherein the probe labeled with a fluorescent intercalative dye alters its fluorescence upon hybridization with the RNA transcript.
